(19) Europäisches Patentamt / European Patent Office / Office européen des brevets

(11) **EP 4 356 721 A1**

(12) **EUROPEAN PATENT APPLICATION**

(43) Date of publication:
24.04.2024 Bulletin 2024/17

(51) International Patent Classification (IPC):
*A01G 7/00* (2006.01)  *G01N 33/00* (2006.01)

(21) Application number: 23158322.0

(52) Cooperative Patent Classification (CPC):
A01G 7/00; G01N 33/0098

(22) Date of filing: 23.02.2023

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 19.10.2022 EP 22202632

(71) Applicant: Krebs Paysagistes SA
1807 Blonay (CH)

(72) Inventors:
• GALLINELLI, Peter
  CH-1217 Mayrin (CH)
• AMOS, Éric
  CH-1225 Chêne-Bourg (CH)
• PALMAN, Marie
  CH-1241 Puplinge (CH)
• RAYBAUD, Blaise
  F-74700 Sallanches (FR)
• KREBS, Stéphane
  CH-1807 Blonay (CH)

(74) Representative: ABREMA SA
Avenue du Théâtre 16
1005 Lausanne (CH)

(54) **APPARATUS FOR MONITORING A HEALTH STATUS OF A TREE**

(57) There is described an apparatus (100) for monitoring a health status of a tree (TR) comprising sensors (10A, 10B) configured to perform measurements of parameters indicative of the health status of the tree (TR) and a processing system (110, 115) configured to process the measurements provided by the sensors (10A, 10B). The sensors (10A, 10B) include at least a multispectral sensor (10A) to carry out optical measurements on a foliage (FO) of the tree (TR), which multispectral sensor (10A) is capable of taking optical measurements in the visible spectrum and the near infrared (NIR) spectrum. The apparatus (100) is configured to compute a Normalized Difference Vegetation Index (NDVI) of the relevant tree (TR) based on the optical measurements carried out by the multispectral sensor (10A).

Fig. 2

Processed by Luminess, 75001 PARIS (FR)

## Description

TECHNICAL FIELD

The present invention generally relates to an apparatus for monitoring a health status of a tree.

BACKGROUND OF THE INVENTION

International (PCT) Publication No. WO 2022/049552 A1 in the name of the present Applicant, the content of which publication is incorporated herein by reference in its entirety, discloses a method and system for tracking, monitoring and predicting the health of a plant heritage, such as a tree park or forest including multiple specimens of trees. Each specimen constituting the plant heritage is equipped with a set of sensors configured to measure a plurality of parameters representative of a health status of the specimen or susceptible to affect the health status of the specimen. Measurement data generated by each set of sensors is gathered locally and communicated to a data collection center via a wireless network. A characterization of the health status of each specimen is performed using the measurement data collected by the data collection center in order to build a predictive model of the health of each specimen. An assessment of the health status of each specimen is undertaken on the basis of the predictive model, and an alert is generated if the predictive model of the health of the specimen is indicative of a danger or another risk related to a worsening of the health of the specimen.

European Patent Publication No. EP 3 531 367 A1 discloses a system for predicting occurrence of a tree disease and a method therefor. The system comprises a storage unit for receiving and storing first moisture information of a tree, which is measured by a dedicated moisture measuring sensor disposed on the tree, and second moisture information of the surrounding soil in which the tree is planted, which is measured by another dedicated moisture measurement sensor disposed in the surrounding soil. Further sensors may be provided to measure humidity and temperature of the environment in which the tree is located, as well as to measure sugar content information about the tree. A processing unit is further provided for predicting whether a tree disease is to occur on the basis of the first moisture information and second moisture information. This publication does not however contain an elaborate description of the sensor technology being used, and merely refers to measurement of the moisture content by a heat pulse and measurement of the sugar content by near-infrared spectroscopy.

European Patent Publication No. EP 3 421 988 A1 discloses an apparatus and method for continuously measuring the "staticity" of one or more trees, namely, the stability thereof over time. The device in essence comprises a triple-axis movement sensor (such as a three-axis accelerometer), a data processing unit, a data transmission unit, and a data receiving unit that are operatively connected to one another. An anemometer may further be connected operatively to the data receiving unit.

Chinese Patent Publication No. CN 103903400 A discloses an apparatus comprising an inclinometer (such as a three-axis MEMS accelerometer) that can be attached to a trunk of a tree to collect real-time acceleration data that is processed to determine a degree of inclination of the tree and compare it to a preset threshold value in order to monitor and determine if the tree is susceptible to fall and constitute a potential risk for bystanders.

A similar principle is disclosed in "Tree tilt monitoring in rural and urban landscapes of Hong Kong using smart sensing technology", Sawaid Abbas et al., published in "Trees, Forest and People", Volume 2, December 2020, 100030, Elsevier B.V. (https://doi.org/10.1016/j.tfp.2020.100030). In this case, an accelerometer and a vibration sensor are used to track the physical response of trees by measuring rotational angles, tree displacements and tree tilt angles within a tilt accuracy of 0.05°.

Yet another principle is disclosed in "IoT-Based Smart Tree Management Solution for Green Cities", Bilal Shabandri et al., published in "Internet of Things and Analytics for Agriculture", Volume 2, Studies in Big Data (SBD) 67, October 2019, pp. 181-199 (https://doi.org/10.1007/978-981-15-0663-5_9). In this case, a variety of sensors are implemented, including a sound sensor, a piezoelectric vibration sensor, a photodetector, a temperature and humidity sensor, an air quality sensor, a moisture sensor and a carbon dioxide sensor, leading to a somewhat complex and costly sensory architecture which is moreover costly and difficult to implement in practice, especially in a portable/mobile solution.

Focussing solely on the physical response of trees and measurement of physical parameters such as tilt angles and tree movements may not provide a sufficiently representative indication of the health status of a tree. It may reliably detect and possibly predict the risk of a tree falling but can hardly be exploited to precisely determine the health status of the tree and build a more reliable predictive model thereof.

As taught in the art, it is therefore preferable to further rely on other sensors to measure parameters other than purely physical parameters relating to stability and/or tree movements, but this comes at the expense of a more complex sensory architecture which becomes even more difficult to implement and integrate in a reliable and cost-effective solution that, by essence, needs to be portable and autonomous from a power supply perspective.

Article titled "New tree monitoring systems: from Industry 4.0 to Nature 4.0", Riccardo Valentini et al., published in "Annals of Silvicultural Research", Volume 43, No. 2, November 30, 2019, pp. 84-89 (https://doi.org/10.12899/asr-1847) discloses an apparatus according to the preamble of claim 1 (see also Italian Patent Publication No. IT 102019000013362 A1 titled "DISPOSITIVO E SISTEMA PER IL RILIEVO DELLO STATO DI SALUTE DI UNA O PIU' PIANTE"). The apparatus (referred to by the name TreeTalker, or "TT" acronym) comprises multispectral sensors to carry out optical measurements on a foliage of the tree, which multispectral sensors are designed to take optical measurements in twelve spectral bands centered at about 450 nm, 500 nm, 550 nm, 570 nm, 600 nm, 610 nm, 650 nm, 680 nm, 730 nm, 760 nm, 810 nm and 860 nm, which allows e.g. for remote computation of a Normalized Difference Vegetation Index (NDVI). The aforementioned TT apparatus is not a such configured to carry out onboard computation of the Normalized Difference Vegetation Index (NDVI), but essentially to communicate the optical measurements (as well as other measurements) in semi-real time via a wireless communication unit configured to operate according to the LoRaWAN communication protocol. In effect, measurement data is transmitted to a nearby node (or "TT-node") that can serve a cluster of multiple TT apparatuses, and data transmission is typically set at hourly frequency. The TT-node is in turn connected to the internet via a GPRS network to send the data to a computer server where it is collected. Such data can then be retrieved from the computer server to carry out processing thereof and extract information indicative of the health status of the trees on which the TT apparatuses are installed.

The aforementioned TT apparatus includes further sensors to measure sap flow, stem humidity, tree trunk radial growth, tree trunk axis movement, as well as air temperature and humidity. Sap flow density is especially retrieved by monitoring the temperature of two 20 mm long probes that are inserted into the stem wood at 10 cm distance along the trunk vertical axis. This is not desirable in practice as this constitutes an invasive measurement that can in effect be harmful to the health of the tree.

Another limitation of the aforementioned TT apparatus resides in the location of the multispectral sensors, which are mounted on top of the main housing of the TT apparatus, and the positioning of the main housing of the TT apparatus which is secured directly to the tree trunk my means of a belt tightened around the tree trunk. This greatly restricts the ability to position the TT apparatus and ensure that the relevant multispectral sensors can properly detect light transmitted through the tree canopy, inherently affecting reliability of the optical measurements. This also imposes restrictions as to the placement of a solar panel used to recharge the apparatus' battery, both of which are located in a separate housing that is attached and electrically connected to the apparatus' main housing.

There therefore remains a need for an improved solution.

SUMMARY OF THE INVENTION

A general aim of the invention is to provide an apparatus for monitoring a health status of a tree that obviates the problems and limitations of the known solutions.

More specifically, an aim of the invention is to provide such an apparatus which provides a good compromise between the representativeness of the measurements and the robustness and cost-effectiveness of the required implementation.

A further aim of the invention is to provide such an apparatus that can reliably carry out representative measurements of a health status of a tree.

Yet another aim of the invention is to provide such an apparatus that can successfully and reliably be implemented in a portable, easily deployable solution.

These aims are achieved thanks to the solutions defined in the claims.

In accordance with the invention, there is provided an apparatus for monitoring a health status of a tree, the features of which are recited in claim 1, namely, such an apparatus comprising sensors configured to perform measurements of parameters indicative of the health status of the tree and a processing system configured to process the measurements provided by the sensors. The sensors include at least a multispectral sensor to carry out optical measurements on a foliage of the tree, which multispectral sensor is capable of taking optical measurements in the visible spectrum and the near infrared (NIR) spectrum. According to the invention, the apparatus is configured to compute a Normalized Difference Vegetation Index (NDVI) of the relevant tree based on the optical measurements carried out by the multispectral sensor.

In one embodiment, the multispectral sensor includes a first sensing device capable of taking a first optical measurement in the blue spectrum and/or red spectrum and a second sensing device capable of taking a second optical measurement in the near infrared spectrum. In such case, the Normalized Difference Vegetation Index (NDVI) is preferably computed taking into account a recalibration factor computed on the basis of optical measurements carried out by the first and second sensing devices in substantially the same spectral band. In another embodiment, the multispectral sensor includes a single sensing device capable of taking both a first optical measurement in the blue spectrum and/or red spectrum and a second optical measurement in the near infrared (NIR) spectrum. By way of preference, the multispectral sensor is configured to take the first optical measurement in the blue spectrum, especially at a spectral band centered at about 450 nm, and the apparatus is configured to compute the Normalized Difference Vegetation Index (NDVI) based on the first optical measurement taken in the blue spectrum and on the second optical measurement taken in the near infrared (NIR) spectrum.

In accordance with a preferred embodiment, electronic components of the apparatus, including the sensors and the processing system, are housed within a casing that is attached to the tree to be monitored via an orientable mount supporting the casing, in which case the apparatus is configured such that the casing is orientable with respect to the tree by means of the orientable mount so that the multispectral sensor can be oriented towards a selected portion of the foliage of the tree. In this context, the casing preferentially includes a casing portion that is substantially transparent to a measurement spectrum of the multispectral sensor. Advantageously, the apparatus further comprises an adjustable attachment mechanism secured to a base of the orientable mount for attachment to a trunk or branch of the tree. The adjustable attachment mechanism may especially be extensible to prevent strangulation of the tree over time.

In accordance with another aspect of the invention, the sensors further include a stability sensor to carry out measurements of a stability of the tree, and the apparatus is further configured to compute an indication of the stability of the tree based on the measurements carried out by the stability sensor. The stability sensor may especially include an accelerometer or inclinometer to measure a degree of inclination of the tree and/or monitor a change over time in the degree of inclination of the tree.

In one embodiment, the apparatus may further comprise a trigger sensor, such as an accelerometer or like motion sensor, to detect a sudden movement of the tree. Such trigger sensor may especially be used to wake up the system in the event of a sudden movement of the tree.

By way of preference, the apparatus further comprises a battery to supply power to the apparatus. Such battery may in particular be rechargeable by means of an energy harvesting device, such as a photovoltaic cell. Preferentially, the battery and, if provided, the energy harvesting device, are housed in the same casing, along with other electronic components of the apparatus, including the sensors and the processing system.

In accordance with a particularly preferred embodiment, the apparatus further comprises a communication unit configured to transmit data indicative of the health status of the tree to a remote station, the data transmitted by the communication unit including or being based at least on the Normalized Difference Vegetation Index (NDVI) computed by the apparatus.

In one embodiment, the apparatus is configured to supply power to the sensors via only when measurements are to be carried out and, to this end, preferably further comprises a first switch to selectively supply or cut power to the sensors, individually or collectively. Even more preferably, the apparatus is configured to supply power to the sensors and/or to the communication unit only when measurements are to be carried out, respectively only when data are to be transmitted. In such case, the apparatus preferably further comprises a first switch to selectively supply or cut power to the sensors, individually or collectively, and a second switch to selectively supply or cut power to the communication unit.

The aforementioned communication unit may advantageously be a wireless communication unit configured to wirelessly transmit the data to the remote station, such as a wireless communication unit configured to operate according to the LoRaWAN or NB-IoT communication protocol.

Lastly, in accordance with a further advantageous embodiment, the apparatus may also comprise a datalogger to at least temporarily store data pending transmission thereof via the communication unit. Furthermore, data may optionally be stored on a portable storage medium, such as an SD card.

Further advantageous embodiments of the invention are discussed below.

BRIEF DESCRIPTION OF THE DRAWINGS

Other features and advantages of the present invention will appear more clearly from reading the following detailed description of embodiments of the invention which are presented solely by way of non-restrictive examples and are illustrated by the appended drawings in which:

Figure 1 is a schematic global view of a system for tracking and monitoring the health status of a population of trees that are each equipped with a sensory and data collection unit such as the apparatus of the invention;
Figure 2 is a schematic illustration of a tree equipped with an apparatus according to one embodiment of the invention;
Figure 3 is a photographic illustration of a prototype of an apparatus according to an embodiment of the invention; and
Figure 4 is a schematic functional diagram of key electronic components of an apparatus according to a preferred embodiment of the invention.

DETAILED DESCRIPTION OF EMBODIMENTS OF THE INVENTION

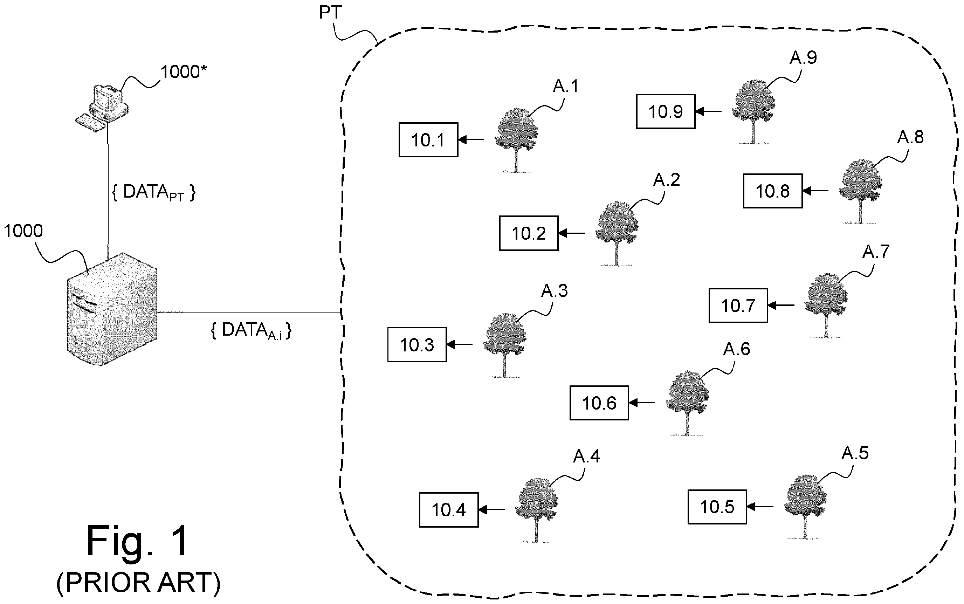

The present invention will be described in relation to various illustrative embodiments as shown in particular in Figures 1 to 4. It shall be understood that the scope of the invention encompasses all combinations and sub-combinations of the features of the invention disclosed herein as defined by the appended claims.

Figure 1 is a schematic global view of a system for tracking and monitoring the health status of a population of trees PT including multiple tree specimens A.i (A.1, A.2, A.3, etc.) that are each equipped with a sensory and data collection unit 10.i (10.1, 10.2, 10.3, etc.). Such a system is generically described in International (PCT) Publication No. WO 2022/049552 A1 previously mentioned in the preamble hereof. The tree specimens A.i are distributed geographically over a defined territory and may indifferently be trees planted in urban and/or rural areas, be it cities, parks, forests, orchards and the like. Each sensory and data collection unit 10.i is configured to permit collection of measurement data, designated $DATA_{A.i}$ indicative of a health status of each tree specimen A.i, which data $DATA_{A.i}$ can appropriately be transmitted to a data collection server 1000 over e.g. a suitable wireless communication network. The collection of data, designated $DATA_{PT}$, thus collected from the multiple data collection points can then be processed on e.g. a remote workstation 1000* to track the health status of the whole population of trees PT, both collectively and individually. The data collection server 1000 may be a cloud server accessible over the Internet, and the remote workstation 1000* may in effect be any suitable workstation 1000*, including a computer station or a smartphone or tablet with access to the data collected by the data collection server 1000.

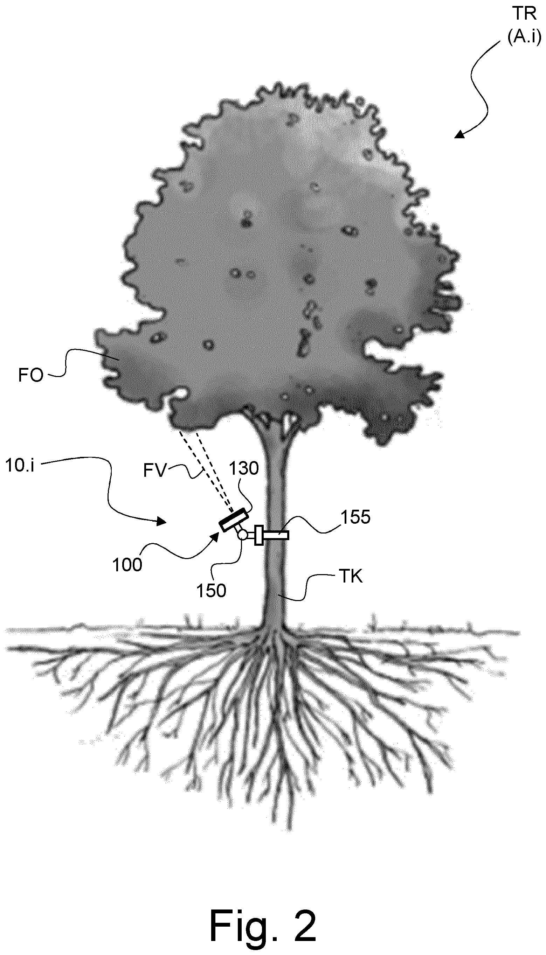

Figure 2 is a schematic illustration of a tree TR, representative of any one of the relevant tree specimens A. i shown in Figure 1, equipped with an apparatus, designated by reference numeral 100, according to one possible embodiment of the invention, which apparatus 100 may act as a suitable sensory and data collection unit 10.i in the context of the system depicted in Figure 1. Apparatus 100 is specifically designed to monitor a health status of the tree TR by, preferably, carrying a combination of measurements, namely, optical measurements on a foliage FO of the tree TR as well as measurements of the stability of the tree TR. As will be described hereafter, apparatus 100 is equipped with dedicated sensors to carry out such measurements, as well as an associated processing system configured to process the measurements. By way of preference, data collected by the apparatus 100 is furthermore transmitted, wirelessly, to a remote station (such as the data collection server 1000 depicted in Figure 1) over a suitable wireless communication network. In the illustrated example, apparatus 100 is preferably attached to a trunk TK of the tree TR by means of a suitable attachment mechanism 155. Even more preferably, apparatus 100 is coupled to the attachment mechanism 155 via an orientable mount 150 so that the apparatus 100 can be appropriately oriented to observe a desired portion of the foliage FO, namely, by orienting a field of view FV of the apparatus 100 towards the desired portion of the foliage FO as schematically depicted. Electronic components of the apparatus 100, including the aforementioned sensors and associated processing system, are suitably housed within a casing 130 that is mounted on the orientable mount 150.

In practice, the apparatus 100 is preferably attached to the tree TR at an elevated position along the trunk TK such as to be out of immediate reach of bystanders, preferably at a height of the order of 2.5 meters to 3 meters.

The orientable mount 150 may be any suitable support mechanism that can be adjusted to change an orientation of the casing 130 and locked in place to ensure that the casing is secured in a stable manner to the associated trunk TK. The orientable mount 150 may especially consist of an articulated linkage with one or more degrees of freedom, including e.g. an adjustable arm mounted on a pivot or ball joint so as to be lockable in one of multiple possible orientations. The adjustable attachment mechanism 155 is preferably extensible so as to prevent strangulation of the tree TR over time, and may especially include an extensible securing belt (and associated tensioning system) wrappable around the trunk TR (or branch) of the tree TR.

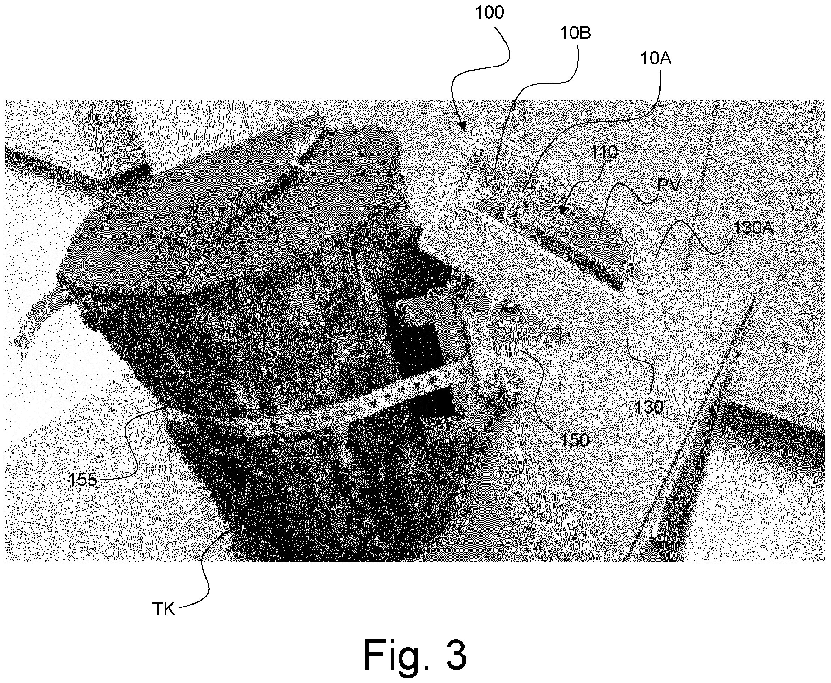

Figure 3 is a photographic illustration of a prototype of an apparatus 100 according to an embodiment of the invention, here shown as being attached to a sawed section of a trunk TK. Visible in Figure 3 is the attachment mechanism 155 including, in the illustrated example, an extensible belt wrapped around the trunk TK, the orientable mount 150 supported via a base thereof onto the attachment mechanism 155, as well as the casing 130 housing the electronic components of the apparatus 100, including an electronic module 110 carrying sensors 10A, 10B.

As this will be appreciated hereafter, the electronic module 110 includes at least a multispectral sensor, designated by reference sign 10A, designed to carry out optical measurements on the foliage FO of the tree TR, and, preferably, a stability sensor, designated by reference sign 10B, designed to carry out measurements of the stability of the tree TR. In that regard, the casing 130 includes a casing portion (here a cover section 130A) that is substantially transparent to a measurement spectrum of the multispectral sensor 10A. While Figure 3 shows the provision of a substantially transparent cover section 130A, one will appreciate that the casing 130 may alternatively be provided with one or more dedicated windows positioned along the field of view FV of the multispectral sensor 10A.

In the illustrated example, the electronic module 110 is further provided with a photovoltaic cell PV acting as energy harvesting device to provide power to recharge a battery (not shown) of the apparatus 100. In that regard, the photovoltaic cell PV may be located within the casing 130, as shown, protected by the cover section 130A, or may alternatively be positioned on an outer portion of the casing 130.

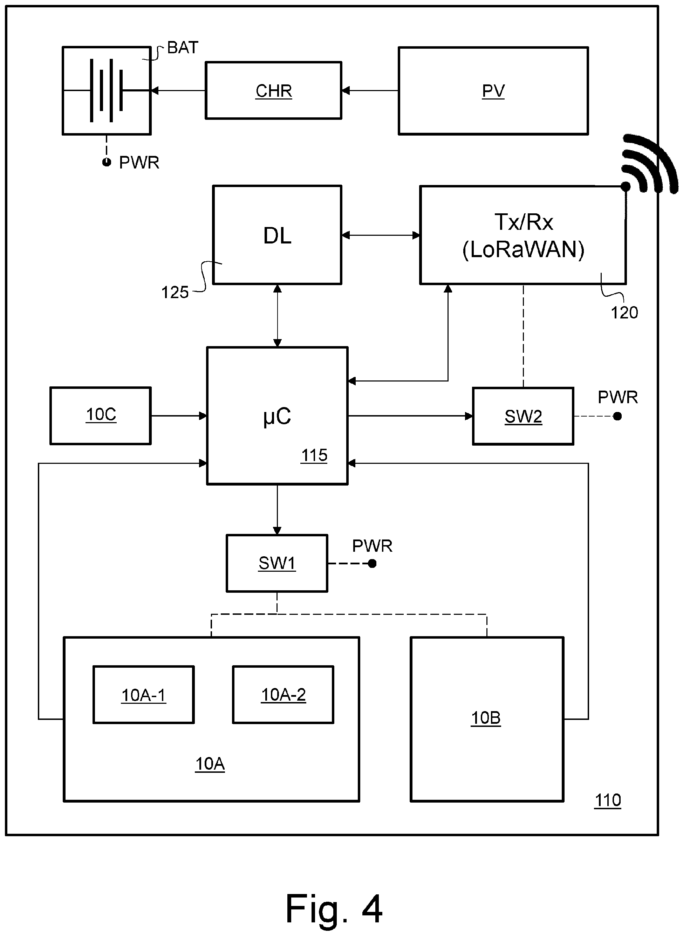

Figure 4 is a schematic functional diagram of key electronic components of the apparatus according to a preferred embodiment of the invention. In the illustrated example, all key electronic components are advantageously provided on a common electronic module 110 comprising:

- a microcontroller or microprocessor 115 handling most of the data processing;
- the multispectral sensor 10A (here including first and second sensing devices 10A-1, 10A-2);

- the stability sensor 10B;
- a trigger sensor 10C;
- a first switch SW1 to control power supply to the sensors 10A, 10B;
- a wireless communication unit 120;
- a second switch SW2 to control power supply to the wireless communication unit 120;
- a datalogger 125;
- a rechargeable battery BAT;
- a charging circuit CHR to control the charge of the battery BAT; and
- the photovoltaic cell PV.

The implementation of all relevant electronic components of the apparatus 100 on a same PCB module 110 brings benefits in terms of reliability and production cost effectiveness compared to the known solutions relying e.g. on the use of multiple interconnected devices.

Additional sensors might be provided in case of need, including sensors capable of measuring representative parameters of the environment in which the tree TR is planted, e.g. temperature and humidity of the environment. Referring to the disclosure of International (PCT) Publication No. WO 2022/049552 A1, the content of which is incorporated herein by reference, such further sensors might be positioned at other desired locations on or next to the tree TR, including e.g. in the soil, next to the roots of the tree TR to measure soil humidity. In such case, the additional sensors might have to be located remotely from the casing 130 housing the key electronics components of the apparatus 100, and operative coupling with the electronic module 110 may be ensured by a suitable wired connection to each remote sensor or via a wireless local interconnection between the electronic module 110 and each remote sensor. Such additional sensors might in effect be provided at any desired location, including e.g. on the orientable mount 150 or the base thereof.

The aforementioned wireless communication unit 120 is preferably a wireless communication unit operating according to the LoRaWAN (long range wide area network) communication protocol, which typically allows for wireless communication over a range of the order of 5 to 10 km with a relatively low power consumption (assuming a corresponding and adequate network coverage is present in practice). Suitable LoRa modules are available on the market from company Semtech Corporation (https://www.semtech.com/).

Other, low-power communication protocols may be contemplated, including the NB-IoT (Narrowband Internet of Things) communication protocol. LoRaWAN and NB-IoT protocols preferably come into consideration in that each such protocols provides for the establishment of a low-power wide-area network permitting wireless communication of data over a relatively long range (namely about 5 to 15 km depending on the technology) and this with low power requirements, which is key to ensuring viability of the solution that must, by essence, be portable and easily deployable over a potentially large area.

It may also be contemplated to transmit data wirelessly over any suitable cellular network (such as over a GSM or GPRS network) or via a suitable satellite uplink.

The switches SW1, SW2, which may consist of simple MOS-FET switches, are designed to allow power to be selectively supplied to or cut from the associated sensors 10A, 10B (here collectively) and communication unit 120, respectively. More specifically, it is advantageously contemplated to supply power to the sensors 10A, 10B and to the communication unit 120 only when measurements are to be carried out and, respectively, only when data are to be transmitted, which is key to ensuring that the apparatus 100 can suitably operate on power provided by the battery BAT. In that regard, the photovoltaic cell PV helps increasing the power autonomy of the apparatus 100 to remain in operation for long durations as might be required to ensure proper monitoring of the health status of the tree TR, namely, over several months or even years.

In other words, the apparatus 100 is preferably configured in such a way as to be put into a low-power sleep mode at times where no measurements are required, in order to save power, and be periodically woken up for the purpose of carrying out the desired measurements. The frequency at which measurements are carried out may vary depending on practical considerations and requirements. By way of illustration, the apparatus 100 may for instance be woken up every ten minutes to carry out and/or transmit the relevant measurements, but a higher or lower frequency of operation may be contemplated. The frequency of operation may also be decreased in the event of a low battery charge status.

As data may be sent only on a periodic basis, the datalogger 125 is provided to ensure temporary storage of the data (be it raw measurement data from the sensors 10A, 10B or processed data as processed by the microcontroller/microprocessor 115) pending transmission thereof via the communication unit 120. This datalogger 125 is also there to ensure that data is not lost in the event of a power failure. In that regard, one may further contemplate the storage of data on a suitable portable storage medium, such as an SD card or the like.

The main purpose of trigger sensor 10C is to detect a sudden movement of the tree TR, which may be triggered by critical factors such as the sudden fall of the tree TR or any other critical event affecting the structural integrity of the tree TR. Trigger sensor 10C may in particular consist of e.g. a motion sensor such as a three-axis accelerometer as for instance marketed by company STMicroelectronics (https://www.st.com/) under product reference LIS2DH12, which is an ultra-low power high-performance three-axis linear accelerometer. Trigger sensor 10C is mainly used here to wake up the system in the event of a sudden movement of the tree TR. Measures could furthermore be contemplated to provide the apparatus 100 with an alarm device to produce an audible alarm in the event the trigger sensor 10C detects a sudden movement of the tree TR.

According to the invention, the multispectral sensor 10A is designed to carry out optical measurements on the foliage FO of the tree TR - as schematically depicted in Figure 2 - and the apparatus 100 is configured to compute a Normalized Difference Vegetation Index (abbreviated "NDVI") of the relevant tree TR based on such optical measurements. More specifically, the multispectral sensor 10A is capable of taking optical measurements in the visible spectrum and the near infrared (NIR) spectrum.

The rationale underlying the NDVI is that live green plants (including trees) absorb solar radiation in the photosynthetically active radiation (PAR) spectral region (i.e. in the wavelength band ranging approximately from 400 to 700 nm), which the live green plants use as a source of energy in the process of photosynthesis. Part of this energy is reemitted in the near infrared (NIR) spectral region (i.e. in the wavelength band ranging from 700 to 1'100 nm). In other words, live, healthy green plants appear relatively dark in the PAR spectral region and relatively bright in the NIR spectral region. In effect, chlorophyll, the most abundant green pigment found in green plants is predominantly absorbent in the blue and red spectra, namely, at or around approximately 450 nm concerning chlorophyll b and at or around approximately 650 nm concerning chlorophyll a.

From an analytical perspective, the NDVI is typically computed as a differential measurement between reflectivity measured in the red spectrum and near infrared (NIR) spectrum in accordance with the following ratio:

$$NDVI = \frac{IR - R}{IR + R}$$

where *IR* is the reflectivity measured in the near infrared spectrum and *R* is the reflectivity measured in the red spectrum. This corresponds to the photosynthesis activity related mostly to chlorophyll a. It is also possible, however, to measure reflectivity in the blue spectrum, which corresponds to the photosynthesis activity related mostly to chlorophyll b, and likewise compute a corresponding NDVI. In such case, the same ratio is calculated, with the main difference residing in *R* designating in such case the reflectivity measured in the blue spectrum.

In one embodiment, as schematically depicted in Figure 4, the multispectral sensor 10A includes a first sensing device 10A-1 that is sensitive to the visible spectrum to take a first optical measurement in the red (or blue) spectrum and a second sensing device 10A-2 that is sensitive to the near infrared (NIR) spectrum to take a second optical measurement in the near infrared (NIR) spectrum. The prototype shown in Figure 3 has been conceived on such basis and use has in particular been made of multispectral sensors as available from company ams-OSRAM AG (https://ams-osram.com/) under product references AS7262 and AS7263. The AS7262 component is a multispectral sensor with sensitivity in the visible spectrum, namely, in six measurement channels (or spectral bands) at and around 450 nm, 500 nm, 550 nm, 570 nm, 600 nm and 650 nm. The AS7263 component is a multispectral sensor with sensitivity in the red and near infrared (NIR) spectra, namely, in six measurement channels (or spectral bands) at and around 610 nm, 680 nm, 730 nm, 760 nm, 810 nm and 860 nm.

By way of preference, the NDVI is calculated in this case with reference to the spectral band centered at 450 nm, i.e. in the blue spectrum, (using the aforementioned AS7262 component) and the spectral band centered at 860 nm (using the aforementioned AS7263 component), tests having demonstrated that this particular selection provides for the best results.

As two distinct multispectral sensors are used in this case, a rebalancing of both sensors has been carried out assuming that optical measurements undertaken with both sensors in the same spectral band should yield the same values. In the present instance, it is assumed that the spectral band centered at 600 nm (using the AS7262 component) and the spectral band centered at 610 nm (using the AS7263 component) should yield substantially the same values.

In other words, considering the illustrative example mentioned above, the NDVI is preferably computed in accordance with the following ratio:

$$NDVI = \frac{R860 - R450 * C}{R860 + R450 * C}$$

where *R860* is the reflectivity measured at approximately 860 nm (using the AS7263 component), *R450* is the reflectivity measured at approximately 450 nm (using the AS7262 component), and *C* is a recalibration factor that is calculated as follows:

$$C = \frac{R610}{R600}$$

where *R610* is the reflectivity measured at approximately 610 nm (using the AS7263 component) and *R600* is the reflectivity measured at approximately 600 nm (using the AS7262 component).

The above example is illustrative of a possible embodiment of the invention that assumes usage of two distinct multispectral components as readily available on the market. It is to be further appreciated that this example takes into account the relevant specifications of each component and the inherent constraints resulting therefrom. Other sensor combinations could be contemplated, in which case the computation methodology might have to be adapted accordingly.

In other embodiments, one could perfectly contemplate use of a single multispectral sensor that exhibits sensitivity in both of the relevant spectral bands, namely, in the blue spectrum and/or red spectrum (e.g. at or around approximately 450 nm and/or 650 nm, respectively), on the one hand, and in the near infrared (NIR) spectrum (e.g. in a selected spectral band from approximately 700 nm to 1'100 nm), on the other hand. In such case, one may advantageously do without any recalibration as a single sensing device is used.

A singularity in the aforementioned approach resides in that the multispectral sensor 10A will typically be oriented upwards towards the foliage FO of the tree TR and that part of the blue sky may in effect be present in the field of view FV of the sensor 10A depending on the relevant density of the foliage FO, which may vary over the year. In other words, assuming a tree with non-persistent foliage changing colour during the fall and disappearing during the winter, reflectivity in the relevant spectral bands will show significant variations over the year, with the NDVI increasing during the spring and summer where foliage FO is the most dense and green, and the NDVI decreasing during the fall and winter where foliage FO is less dense, or even non-existent, and changes colour.

In accordance with the preferred embodiment shown schematically in Figure 4, the sensors further include a stability sensor 10B to carry out measurements of a stability of the tree TR, and the apparatus 100 is further configured to compute an indication of the stability of the tree TR based on the measurements carried out by the stability sensor 10B. Such stability sensor 10B may in particular include an accelerometer or inclinometer to measure a degree of inclination of the tree TR and/or monitor a change over time in the degree of inclination of the tree TR. By way of illustration, the prototype shown in Figure 3 has been conceived on the basis of a three-axis high-precision inclinometer as available from company muRata (https://www.murata.com/) under product reference SCL3300. Such inclinometer is capable of providing a measurement of angles of inclination with an output resolution of 0.0055°/LSB.

In the present example, stability sensor 10B is configured to measure a movement of the apparatus 100 (which is assumed to be fixedly secured to the tree TR and thus follows movement thereof). A calibration is performed after mounting of the apparatus 100 in the relevant, final position on the tree in order to obtain a reference value for subsequent comparison. If the tree TR moves, yielding a corresponding movement of the apparatus 100, and thus of the stability sensor 10B, a corresponding change is detected by the inclinometer 10B, which allows in turn to compute an indication of the change over time of the degree of inclination of the tree TR. In effect, by adequate processing of the information provided by the inclinometer 10B, one can determine the degree of inclination and any change thereof, as well as, potentially, the direction in which the tree TR is moving. As the only force applied on the inclinometer 10B is that generated by gravity (assuming that the tree remains stable during the measurement), a rotational movement about the relevant, vertical axis may not cause modification of the relevant measurement components of the inclinometer 10B. It may therefore be difficult to determine the direction of movement of the tree TR with high accuracy, and only a gross indication of such direction of movement might potentially be determined in practice. This being said, such gross indication is nevertheless sufficient to determine and rate the risk of the tree TR falling onto a specified zone, such as a neighbouring road or footpath.

Various modifications and/or improvements may be made to the above-described embodiments without departing from the scope of the invention as defined by the appended claims.

LIST OF REFERENCE NUMERALS AND SIGNS USED THEREIN

| | |
|---|---|
| 100 | apparatus for monitoring health status of tree TR |
| 10A | multispectral sensor |
| 10A-1 | sensing device sensitive to visible spectrum (in particular blue and/or red spectrum) |
| 10A-2 | sensing device sensitive to near infrared (NIR) spectrum |
| 10B | stability sensor (e.g. accelerometer or inclinometer) |
| 10C | trigger sensor (e.g. accelerometer or like motion sensor) |
| 110 | electronic module |
| 115 | microcontroller/microprocessor |
| 120 | transceiver unit (e.g. LoRaWAN unit) |
| 125 | datalogger (DL) |
| 130 | casing |
| 130A | transparent cover |
| 150 | orientable mount |
| 155 | adjustable attachment mechanism |
| BAT | rechargeable battery |
| CHR | battery charging circuit |
| PV | photovoltaic cell |
| SW1 | (first) switch (power supply to sensors 10A, 10B) |
| SW2 | (second) switch (power supply to transceiver unit 120) |
| TR | tree |
| TK | tree trunk |
| FO | foliage of tree TR |
| FV | field of view of multispectral sensor 10A |
| PT | population of tree specimens A.i being monitored |
| A.i | individual tree specimens |
| 10.i | sensory and data collection unit (e.g. apparatus 100) provided on each tree specimen A.i |
| 1000 | remote server for data collection |

1000*   remote working station for data processing

**Claims**

1.  An apparatus (100) for monitoring a health status of a tree (TR) comprising sensors (10A, 10B) configured to perform measurements of parameters indicative of the health status of the tree (TR) and a processing system (110, 115) configured to process the measurements provided by the sensors (10A, 10B),

    wherein the sensors (10A, 10B) include at least a multispectral sensor (10A) to carry out optical measurements on a foliage (FO) of the tree (TR), which multispectral sensor (10A) is capable of taking optical measurements in the visible spectrum and the near infrared (NIR) spectrum, **characterized in that** the apparatus (100) is configured to compute a Normalized Difference Vegetation Index (NDVI) of the relevant tree (TR) based on the optical measurements carried out by the multispectral sensor (10A).

2.  The apparatus (100) according to claim 1, wherein the multispectral sensor (10A) includes a first sensing device (10A-1) capable of taking a first optical measurement in the blue spectrum and/or red spectrum and a second sensing device (10A-2) capable of taking a second optical measurement in the near infrared (NIR) spectrum, and wherein the Normalized Difference Vegetation Index (NDVI) is preferably computed taking into account a recalibration factor computed on the basis of optical measurements carried out by the first and second sensing devices (10A-1, 10A-2) in substantially the same spectral band.

3.  The apparatus (100) according to claim 1, wherein the multispectral sensor (10A) includes a single sensing device capable of taking both a first optical measurement in the blue spectrum and/or red spectrum and a second optical measurement in the near infrared (NIR) spectrum.

4.  The apparatus according to claim 2 or 3, wherein the multispectral sensor (10A) is configured to take the first optical measurement in the blue spectrum, especially at a spectral band centered at about 450 nm, and wherein the apparatus (100) is configured to compute the Normalized Difference Vegetation Index (NDVI) based on the first optical measurement taken in the blue spectrum and on the second optical measurement taken in the near infrared (NIR) spectrum.

5.  The apparatus (100) according to any one of the preceding claims, wherein electronic components of the apparatus (100), including the sensors (10A, 10B) and the processing system (110, 115), are housed within a casing (130) that is attached to the tree (TR) to be monitored via an orientable mount (150) supporting the casing (130), and wherein the apparatus (100) is configured such that the casing (130) is orientable with respect to the tree (TR) by means of the orientable mount (150) so that the multispectral sensor (10A) can be oriented towards a selected portion of the foliage (FO) of the tree (TR).

6.  The apparatus (100) according to claim 5, wherein the casing (130) includes a casing portion (130A) that is substantially transparent to a measurement spectrum of the multispectral sensor (10A).

7.  The apparatus (100) according to claim 5 or 6, further comprising an adjustable attachment mechanism (155) secured to a base of the orientable mount (150) for attachment to a trunk (TK) or branch of the tree (TR), wherein the adjustable attachment mechanism (155) is preferably extensible to prevent strangulation of the tree (TR) over time.

8.  The apparatus (100) according to any one of the preceding claims, wherein the sensors (10A, 10B) further include a stability sensor (10B) to carry out measurements of a stability of the tree (TR),

    wherein the apparatus (100) is further configured to compute an indication of the stability of the tree (TR) based on the measurements carried out by the stability sensor (10B), and wherein the stability sensor (10B) preferably includes an accelerometer or inclinometer to measure a degree of inclination of the tree (TR) and/or monitor a change over time in the degree of inclination of the tree (TR).

9.  The apparatus (100) according to any one of the preceding claims, further comprising a trigger sensor (10C), such as an accelerometer or like motion sensor, to detect a sudden movement of the tree (TR).

10. The apparatus (100) according to any one of the preceding claims, further comprising a battery (BAT) to supply power to the apparatus (100),

    wherein the battery (BAT) is preferably rechargeable by means of an energy harvesting device, such as a photovoltaic cell (PV), and wherein the battery (BAT) and, if provided, the energy harvesting device, are preferentially housed in the same casing (130), along with other electronic components of the apparatus (100), including the sensors (10A, 10B) and the processing system (110, 115).

11. The apparatus (100) according to any one of the preceding claims, further comprising a communication unit (120) configured to transmit data indicative of the health status of the tree (TR) to a remote station (1000, 1000*),
and wherein the data transmitted by the communication unit (120) includes or is based at least on the Normalized Difference Vegetation Index (NDVI) computed by the apparatus (100).

12. The apparatus (100) according to any one of the preceding claims, wherein the apparatus (100) is configured to supply power to the sensors (10A, 10B) only when measurements are to be carried out,
and wherein the apparatus (100) preferably further comprises a first switch (SW1) to selectively supply or cut power to the sensors (10A, 10B), individually or collectively.

13. The apparatus (100) according to claim 11, wherein the apparatus (100) is configured to supply power to the sensors (10A, 10B) and/or to the communication unit (120) only when measurements are to be carried out, respectively only when data are to be transmitted,
and wherein the apparatus (100) preferably further comprises a first switch (SW1) to selectively supply or cut power to the sensors (10A, 10B), individually or collectively, and a second switch (SW2) to selectively supply or cut power to the communication unit (120).

14. The apparatus (100) according to claim 11 or 13, wherein the communication unit (120) is a wireless communication unit (120) configured to wirelessly transmit the data to the remote station (1000, 1000*), and wherein the wireless communication unit (120) is preferably configured to operate according to the LoRaWAN or NB-IoT communication protocol.

15. The apparatus (100) according to any one of claims 11, 13 and 14, further comprising a datalogger (125) to at least temporarily store data pending transmission thereof via the communication unit (120),
and wherein data is optionally stored on a portable storage medium, such as an SD card.

Fig. 1
(PRIOR ART)

Fig. 2

Fig. 3

Fig. 4

Europäisches
Patentamt

European
Patent Office

Office européen
des brevets

# EUROPEAN SEARCH REPORT

Application Number

EP 23 15 8322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Riccardo Valentini: "The TreeTalker network: let's trees talk about climate", , 27 February 2019 (2019-02-27), XP055681057, Retrieved from the Internet: URL:https://www.slideshare.net/lifeurbangreen/the-treetalker-network-lets-trees-talk-about-climate [retrieved on 2020-03-30] * Please note that the internet link provides 17 pages of 'slides' related to the Tree Talker apparatus.; page 1 - page 17 * | 1,3,5-15 | INV. A01G7/00 G01N33/00 |
| X A | EP 3 473 081 A1 (SONY CORP [JP]) 24 April 2019 (2019-04-24) * abstract; figures 1-29 * * paragraph [0014] - paragraph [0158] * | 1,2 3-15 | |
| X A | WO 2016/181743 A1 (KONICA MINOLTA INC [JP]) 17 November 2016 (2016-11-17) * The corresponding text related to figure 5 in the description explains the apparatus and its functioning.; figure 5 * | 1 2-15 | TECHNICAL FIELDS SEARCHED (IPC) A01G G01N |
| X A | JP 2022 087038 A (TOKYO KOGEI UNIV) 9 June 2022 (2022-06-09) * the whole document * | 1,4 2,3,5-15 | |
| A,D | IT 2019 0001 3362 A1 (NATURE 4 0 SB S R L [IT]) 30 January 2021 (2021-01-30) * the whole document * | 1-15 | |
| A | WO 2019/069219 A1 (PNAT S R L [IT]) 11 April 2019 (2019-04-11) * the whole document * | 1-15 | |

The present search report has been drawn up for all claims

| Place of search | Date of completion of the search | Examiner |
|---|---|---|
| The Hague | 17 August 2023 | Balzar, Maarten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................................
& : member of the same patent family, corresponding document

EPO FORM 1503 03.82 (P04C01)

**EP 4 356 721 A1**

**ANNEX TO THE EUROPEAN SEARCH REPORT
ON EUROPEAN PATENT APPLICATION NO.**

**EP 23 15 8322**

This annex lists the patent family members relating to the patent documents cited in the above-mentioned European search report.
The members are as contained in the European Patent Office EDP file on
The European Patent Office is in no way liable for these particulars which are merely given for the purpose of information.

**17-08-2023**

| Patent document cited in search report | | Publication date | Patent family member(s) | | Publication date |
|---|---|---|---|---|---|
| EP 3473081 | A1 | 24-04-2019 | CN 109310053 | A | 05-02-2019 |
| | | | EP 3473081 | A1 | 24-04-2019 |
| | | | JP 7001969 | B2 | 04-02-2022 |
| | | | JP WO2017217258 | A1 | 11-04-2019 |
| | | | US 2019293559 | A1 | 26-09-2019 |
| | | | WO 2017217258 | A1 | 21-12-2017 |
| WO 2016181743 | A1 | 17-11-2016 | CN 107532997 | A | 02-01-2018 |
| | | | JP 7088277 | B2 | 21-06-2022 |
| | | | JP 2021056236 | A | 08-04-2021 |
| | | | JP WO2016181743 | A1 | 01-03-2018 |
| | | | KR 20170133505 | A | 05-12-2017 |
| | | | KR 20190085181 | A | 17-07-2019 |
| | | | WO 2016181743 | A1 | 17-11-2016 |
| JP 2022087038 | A | 09-06-2022 | NONE | | |
| IT 201900013362 | A1 | 30-01-2021 | | | |
| WO 2019069219 | A1 | 11-04-2019 | AU 2018344597 | A1 | 23-04-2020 |
| | | | BR 112020006837 | A2 | 06-10-2020 |
| | | | CA 3078108 | A1 | 11-04-2019 |
| | | | CN 111316075 | A | 19-06-2020 |
| | | | EP 3692345 | A1 | 12-08-2020 |
| | | | JP 2021500538 | A | 07-01-2021 |
| | | | US 2020240832 | A1 | 30-07-2020 |
| | | | WO 2019069219 | A1 | 11-04-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2022049552 A1
- EP 3531367 A1
- EP 3421988 A1
- CN 103903400 A
- IT 102019000013362 A1

### Non-patent literature cited in the description

- Tree tilt monitoring in rural and urban landscapes of Hong Kong using smart sensing technology. **SAWAID ABBAS et al.** Trees, Forest and People. Elsevier B.V, December 2020, vol. 2, 100030
- **BILAL SHABANDRI et al.** IoT-Based Smart Tree Management Solution for Green Cities. *Internet of Things and Analytics for Agriculture,* October 2019, vol. 2, 181-199, https://doi.org/10.1007/978-981-15-0663-5_9
- **RICCARDO VALENTINI et al.** New tree monitoring systems: from Industry 4.0 to Nature 4.0. *Annals of Silvicultural Research,* 30 November 2019, vol. 43 (2), 84-89, https://doi.org/10.12899/asr-1847